(19) Europäisches Patentamt / European Patent Office / Office européen des brevets

(11) **EP 2 670 827 B1**

(12) **FASCICULE DE BREVET EUROPEEN**

(45) Date de publication et mention de la délivrance du brevet:
**16.01.2019 Bulletin 2019/03**

(21) Numéro de dépôt: **12702238.2**

(22) Date de dépôt: **31.01.2012**

(51) Int Cl.:
*C11B 3/14* (2006.01)  *A61K 8/31* (2006.01)
*A61K 8/63* (2006.01)  *C07D 311/72* (2006.01)
*A61K 8/67* (2006.01)  *A61Q 19/06* (2006.01)
*A61Q 19/08* (2006.01)  *A61K 8/97* (2017.01)
*A61K 31/20* (2006.01)  *A61K 31/355* (2006.01)
*A61K 31/56* (2006.01)

(86) Numéro de dépôt international:
**PCT/EP2012/051602**

(87) Numéro de publication internationale:
**WO 2012/104319 (09.08.2012 Gazette 2012/32)**

(54) **UTILISATION D'AU MOINS UN CO-PRODUIT DE L'INDUSTRIE DU RAFFINAGE DES HUILES VÉGÉTALES POUR OBTENIR UN INSAPONIFIABLE TOTAL PURIFIÉ D'HUILE VÉGÉTALE**

VERWENDUNG VON MINDESTENS EINEM NEBENPRODUCT AUS DER RAFFINIERUNG VON PFLANZENÖL ZUR GEWINNUNG EINES GEREINIGTEN NICHT VERSEIFBAREN PFLANZENÖLPRODUKTS

USE OF AT LEAST ONE COPRODUCT FROM THE VEGETABLE-OIL REFINING INDUSTRY FOR OBTAINING A PURIFIED TOTAL UNSAPONIFIABLE VEGETABLE OIL PRODUCT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.01.2011 FR 1150742**
**31.01.2011 US 201161438023 P**

(43) Date de publication de la demande:
**11.12.2013 Bulletin 2013/50**

(73) Titulaire: **Laboratoires Expanscience**
**92048 Paris La Défense Cedex (FR)**

(72) Inventeurs:
• **LEGRAND, Jacques**
**61290 Neuilly sur Eure (FR)**
• **SAUNOIS, Alex**
**28100 Dreux (FR)**
• **BAUDOUIN, Caroline**
**78120 Rambouillet (FR)**
• **MSIKA, Philippe**
**78000 Versailles (FR)**

(74) Mandataire: **Regimbeau**
**20, rue de Chazelles**
**75847 Paris Cedex 17 (FR)**

(56) Documents cités:
**US-A- 2 349 270**    **US-A- 5 512 691**
**US-A- 5 702 714**    **US-A1- 2004 030 166**
**US-A1- 2005 250 953**

• **FANG ET AL: "Separation of natural tocopherols from soybean oil byproduct with supercritical carbon dioxide", JOURNAL OF SUPERCRITICAL FLUIDS, PRA PRESS, US, vol. 40, no. 1, 16 décembre 2006 (2006-12-16), pages 50-58, XP005731194, ISSN: 0896-8446, DOI: 10.1016/J.SUPFLU.2006.04.008**
• **JIANG ET AL: "Molecular Distillation for recovering Tocopherol and Fatty Acid Methyl Esters from Rapeseed Oil Deodoriser Distillate", BIOSYSTEMS ENGINEERING, ACADEMIC PRESS, UK, vol. 93, no. 4, 1 avril 2006 (2006-04-01), pages 383-391, XP005358766, ISSN: 1537-5110, DOI: 10.1016/J.BIOSYSTEMSENG.2006.01.008**

**Description**

La présente invention concerne l'utilisation d'au moins un co-produit de l'industrie du raffinage des huiles végétales pour obtenir un insaponifiable total purifié d'huile végétale débarrassé des impuretés initialement présentes dans ledit co-produit, avantageusement débarrassé des composés sapides et/ou odorants et/ou des composés chimiques résultant de l'altération et de la dégradation des huiles végétales. L'invention concerne également un procédé d'obtention d'un insaponifiable total purifié d'huile végétale à partir d'au moins un coproduit de l'industrie du raffinage des huiles végétales.

L'insaponifiable constitue la fraction d'un corps gras qui, après action prolongée d'une base alcaline, reste insoluble dans l'eau et peut être extraite par un solvant organique.

Les huiles végétales contiennent typiquement entre 0,5 et 2 % d'insaponifiables (Schwartz, 1988 ; Hamilton et Rossel, 1986).

Quatre grands groupes ou familles de substances sont présents dans la plupart des insaponifiables d'huiles végétales. Le groupe le plus important est généralement représenté par celui qui rassemble les stérols incluant les alcools triterpéniques pentacycliques et les 4-méthylstérols. Le second groupe est généralement constitué par les tocophérols pouvant intégrer des tocotriénols. Les deux autres groupes sont les alcools aliphatiques et les hydrocarbures saturés et insaturés.

La composition qualitative et quantitative de ces fractions insaponifiable varie en fonction de la nature des huiles végétales à partir desquelles sont extraits les insaponifiables.

De nombreuses activités biologiques ont été attribuées à ces composés. Ils sont souvent utilisés dans de nombreuses applications dans les domaines de la Pharmacie, de la Cosmétique et de l'Alimentaire.

En particulier, les stérols sont en général utilisés pour leurs propriétés hypocholestérolémiantes, anti-inflammatoires ou antivieillissement. Les tocophérols, plus souvent dénommés Vitamine E et tocotriénols, sont des antioxydants naturels reconnus pour leurs effets antioxydants in-vivo et in-vitro, ainsi que pour leurs propriétés vitaminiques. Le squalène, principal représentant des hydrocarbures, sous sa forme naturelle et hydrogénée présente également des propriétés physico-chimiques particulières qui lui permettent de jouer un rôle dans des formulations pharmaceutiques et cosmétiques.

Généralement, les stérols, les tocophérols ou encore le squalène sont des fractions d'insaponifiables - ou un constituant spécifique des insaponifiables pour ce qui est du squalène - de nature physico-chimique différente qui ont été isolés et purifiés à partir des insaponifiables d'huiles végétales.

Divers procédés ont ainsi été développés pour extraire sélectivement ces fractions d'insaponifiables à partir des huiles végétales. Ces procédés intègrent majoritairement des étapes intermédiaires de concentration, cristallisation fractionnée, précipitation, partage par solvants ou distillation moléculaire, qui visent à limiter les volumes à traiter dans l'étape finale d'extraction liquide/liquide de l'insaponifiable. Cependant, au regard du prix des matières premières, des étapes à mettre en oeuvre et des volumes à traiter, ces procédés ne sont pas économiquement viables.

L'utilisation des sous-produits du raffinage des huiles est donc apparue comme une alternative moins coûteuse pour produire les insaponifiables d'huiles végétales.

En particulier, les distillats de désodorisation ou DD représentent un sous-produit de l'industrie du raffinage des huiles particulièrement riches en insaponifiable. Lors de la dernière étape du raffinage des huiles végétales, une opération de désodorisation est réalisée par injection de vapeur sèche dans l'huile maintenue sous vide à haute température. Il s'agit d'une distillation avec entrainement par la vapeur d'eau des constituants les plus volatils de l'huile, notamment les composés responsables de l'odeur et du goût parfois appelés les « flaveurs », les acides gras libres, mais aussi des substances provenant de la dégradation des huiles et/ou des traces de contaminants.

Les conditions de vide et de température utilisées lors de la désodorisation, couplées à l'injection de vapeur, favorisent l'entrainement d'une partie des constituants de l'insaponifiable de l'huile. Le sous-produit représenté par le condensat des vapeurs de stripage des huiles végétales contient donc une concentration en insaponifiable remarquable et qui justifie sa valorisation dans la filière d'extraction des fractions des insaponifiables.

Suivant les conditions de raffinage choisies et les performances des appareils industriels, ainsi que des systèmes de condensation et de séparation des distillats de désodorisation, des facteurs d'enrichissement en insaponifiable très variables, par rapport à l'huile brute de départ, peuvent être obtenus. Ils se situent entre 5 et 25.

Dans le cas de l'huile de soja par exemple, des teneurs de 20 %, 20 % et 3,5 % respectivement pour les stérols, les tocophérols et le squalène peuvent être obtenues dans les DD.

Il existe ainsi divers procédés d'extraction qui permettent d'isoler et de purifier les tocophérols ou les stérols à partir de co-produits de l'industrie du raffinage des huiles végétales.

On peut citer ainsi la demande FR 2 803 598 de la Demanderesse qui décrit un procédé d'extraction sélective de fractions spécifiques des insaponifiables, telles que les tocophérols (vitamine E) et les stérols, par cristallisation ou extraction liquide/liquide, en utilisant un solvant particulier comme le chloro-1-butane, à partir d'une huile végétale ou d'un co-produit de l'industrie du raffinage des huiles végétales.

On peut encore citer par exemple la demande WO 2010/004193 qui décrit un procédé permettant d'extraire par fractionnement des fractions isolées et purifiées de l'insaponifiable, telles que le squalène, les stérols et la vitamine E, à partir de condensats de raffinage physique et/ou de distillats de désodorisation d'huiles végétales, notamment par distillation et cristallisation. La cristallisation permet notamment d'isoler et purifier les stérols et la distillation fractionnée ou moléculaire permet d'isoler et purifier la Vitamine E.

On peut aussi citer la demande US2004/0030166 qui décrit un procédé de traitement d'un distillat de désodorisation d'huile pour obtenir les stérols et tocophérols.

Ainsi, les procédés d'extraction de l'art antérieur permettent d'extraire des fractions spécifiques purifiées des insaponifiables d'huiles végétales à partir de sous-produits de l'industrie du raffinage des huiles végétales, mais ne permettent pas, en raison des propriétés physico-chimiques des différentes fractions, d'extraire un insaponifiable total purifié, contenant préférentiellement l'ensemble des familles ou constituants naturellement présents dans l'insaponifiable d'une huile donnée.

Or, à côté des constituants propres à l'insaponifiable, d'autres composés tels que des impuretés sont présents dans les co-produits de l'industrie du raffinage des huiles végétales, tels que les DD, qui ne présentent pas de potentiel d'activité intéressant, qui dénaturent le produit ou qui démontrent un facteur de toxicité reconnu. Nous pouvons citer notamment :

- des composés sapides et/ou odorants tels que le deca-2 ,4-diénal-(E,E), l'hexanal, l'hexanol, le 1 hexanol-2-ethyl, les terpènes comme le $\beta$-Caryophyllène ... ;
- des composés chimiques résultant de l'altération et la dégradation des huiles, tels que les aldéhydes, cétones, hydrocarbures légers ...
- des polluants : traces de résidus phytosanitaires tels que les pesticides...
- des contaminants qui peuvent résulter de contaminations croisées (brassicastérol...).

Ainsi, les volumes manipulés sur les unités qui traitent les huiles végétales sont très importants et proviennent de nombreuses origines (palme, soja, tournesol, colza, arachide...). Les mêmes unités peuvent donc traiter alternativement différentes origines d'huiles, ce qui oblige à un contrôle particulier de la traçabilité et du conditionnement des DD fabriqués et à une gestion stricte des nettoyages, mais ce qui génère également des risques de contaminations croisées.

Ainsi, il est très fréquent de détecter dans les DD, et plus particulièrement dans les DD du soja, un composé tel que le brassicastérol. Le brassicastérol est un des constituants caractéristiques de l'huile de colza. Ainsi, une teneur importante en brassicastérol permet de suspecter avec une forte probabilité la présence d'huile de colza. Il s'agit ainsi d'un traceur qui permet de déterminer la pureté des huiles et des insaponifiables, notamment celle du soja, et qui permet d'identifier cette pollution.

Du fait de sa présence systématique sous forme de traces en tant qu'intermédiaire dans le métabolisme des végétaux, le brassicastérol peut être systématiquement détecté dans les insaponifiables, mais la cause la plus récurrente de présence de ce composé dans les insaponifiables produits à partir de DD est l'utilisation d'une matière première issue d'un mélange de différentes huiles, et notamment de l'huile de colza.

Des normes ont été fixées pour contrôler la présence de ce traceur et pour garantir la pureté des produits préparés. En particulier, le Codex Alimentarius détermine des normes et des recommandations pour la sécurité sanitaire des aliments, alors que la Pharmacopée Européenne fixe elle aussi des normes pour les médicaments.

A titre d'exemple, ces deux organismes accordent leurs évaluations pour limiter à 0,3% des stérols totaux la teneur relative en brassicastérol dans l'insaponifiable d'huile de soja.

Alors que tous les schémas extractifs s'orientent vers la purification de familles spécifiques au regard des propriétés physico-chimiques différentes de ces dernières entre-elles, il existait un besoin de mettre au point un nouveau procédé visant à obtenir l'ensemble des constituants et familles de constituants composant l'insaponifiable d'une huile végétale spécifique à partir de sous-produits de l'industrie du raffinage des huiles, tels que les DD, tout en éliminant sélectivement et de manière substantielle les diverses impuretés présentes dans lesdits sous-produits, sans dégrader qualitativement et quantitativement les différents constituants composant ledit insaponifiable.

La présente invention vient combler ce besoin. La Demanderesse a ainsi découvert un nouveau procédé d'obtention d'un insaponifiable total d'huile végétale, de composition reproductible, garant de son origine, substantiellement débarrassé des diverses impuretés présentes dans les sous-produits de départ, et dans lequel sont conservés les différents constituants de l'insaponifiable sans être dégradés.

Le procédé selon la présente invention est ainsi un procédé d'extraction sélective de l'insaponifiable total d'huile végétale à partir d'un produit de départ contenant de nombreuses impuretés, ce qui était difficile et n'avait pas été réalisé jusqu'alors, compte-tenu du fait que l'insaponifiable total est constitué d'un mélange de composés de natures chimiques très diverses et présentant des propriétés physico-chimiques très différentes.

En outre, la comparaison des compositions des insaponifiables des différentes huiles végétales fait apparaître une variabilité importante sur les teneurs relatives des principaux constituants. Elle met également en évidence la présence de composés spécifiques en relation avec des huiles particulières. Il n'était ainsi pas évident de réussir à extraire l'ensemble des composés et familles de composés constituant les insaponifiables de diverses huiles végétales avec un bon rendement, sans dégradation, tout en conservant la composition de l'insaponifiable de chaque huile d'origine.

La Demanderesse a ainsi découvert un procédé d'extraction répondant à l'ensemble de ces exigences, et en particulier, permettant de conserver au sein de chaque grande famille des insaponifiables la composition relative des composés principaux qui caractérise son huile d'origine. La composition de l'insaponifiable total obtenu selon le procédé objet de la présente invention permet donc de déterminer de quel type d'huile végétale le produit préparé est issu.

Enfin, l'utilisation d'une matière première considérée comme un sous-produit des huileries présente certes une richesse intéressante en insaponifiable comme vu ci-dessus, mais rassemble également de nombreux composés indésirables, odorants, sapides, instables, et toxiques. Tous ces composés indésirables dans les huiles alimentaires raffinées qui sont nuisibles pour la santé le sont tout autant pour les applications des insaponifiables en cosmétique, nutraceutique ou dans le domaine pharmaceutique.

Classiquement, dans les procédés de l'art antérieur visant à purifier les huiles végétales, de tels composés indésirables peuvent être éliminés des huiles végétales au moins en partie par une étape de désodorisation lors du raffinage de l'huile. Une telle étape de désodorisation est généralement réalisée par stripage vapeur dans des conditions extrêmes. Typiquement, on applique un vide de l'ordre de 2 à 4 mbars et une température pouvant atteindre 250°C, sous courant de vapeur, ce qui entraine une perte significative en insaponifiable, démontrant ainsi le caractère non sélectif d'une telle opération.

Par conséquent, l'utilisation d'un procédé identique pour purifier un insaponifiable par désodorisation, dans les mêmes conditions que celles utilisées pour les huiles, ne conduirait pas à une purification sélective de l'insaponifiable, génèrerait des pertes importante de composés valorisables et engendrerait systématiquement une modification de la composition de l'insaponifiable.

Tous les constituants du mélange à purifier ayant des pressions de vapeur très proches, il paraît ainsi difficile d'envisager un fractionnement et une élimination sélective des composés indésirables dans des conditions de vide et de température stabilisées de désodorisation.

Par ailleurs, le procédé de désodorisation par stripage vapeur suit les lois de Raoult et Dalton. A partir de ces lois, Sarkadi (J.A.O.C.S. 35, 472-475, 1958) a proposé une formule de base qui permet de définir l'impact des différents paramètres sur l'efficacité du procédé.

$$n_{vap} = \frac{n_{huile} P^{\circ}}{E P_v} \ln \frac{C_l^0}{C_l}$$

$n_{vap}$ le nombre de moles de vapeur
$n_{huile}$ le nombre de mole d'huile
$P^0$ la pression totale de désodorisation
$P_v$ la pression de vapeur saturante du composé à éliminer
$C_1^{\circ}$ et $C_1$ concentration initiale et finale du composé à éliminer
$E$ coefficient d'efficacité

Il peut ainsi être constaté, à la vue de cette formule, que de nombreux paramètres interviennent et interagissent dans la désodorisation. A partir de cette formule, il apparaît donc que les conditions opératoires de séparation et d'élimination des impuretés sont très difficiles à prévoir, d'autant que le mélange à traiter comporte de multiples composés à éliminer et pour la plupart desquels les caractéristiques de pression de vapeur et les concentrations de départ ne sont pas connues précisément.

D'autre part, le comportement du mélange à traiter lors de la phase d'injection de vapeur est directement lié à la pression et à la température, ainsi qu'au débit de vapeur qui, s'il est mal ajusté, provoque l'entrainement vésiculaire, voire l'emballement, de la distillation lors des phases de fractionnement.

Une alternative à la désodorisation pourrait être représentée par la distillation fractionnée, mais ce type d'opération de génie chimique réalisée en batch nécessite des températures et des temps de chauffage importants qui sont préjudiciables à la stabilité des composants fragiles de l'insaponifiable, et notamment des tocophérols.

Une autre alternative pourrait être la distillation moléculaire, mais au regard du faible pouvoir séparateur de cette technologie, la distillation moléculaire ne présentait pas un intérêt particulier pour résoudre le problème qui est posé.

Face à ces états de fait, un des objectifs principaux de l'invention était de proposer une méthode permettant d'éliminer sélectivement les impuretés des insaponifiables totaux extraits des co-produits de l'industrie du raffinage des huiles.

La Demanderesse a ainsi découvert un nouveau procédé d'obtention d'un insaponifiable total d'huile végétale permettant de résoudre ce problème et de pallier les inconvénients des techniques antérieures, avantageusement par l'utilisation d'opérations de stripage par un gaz vecteur sous vide dans des conditions de maîtrise parfaite d'un gradient de température et de vide dans l'installation.

De manière tout particulièrement avantageuse, le procédé ou l'utilisation selon l'invention inclut notamment une étape d'élimination substantielle des impuretés potentiellement toxiques de types composés sapides et/ou odorants et/ou composés chimiques résultant de l'altération et de la dégradation des huiles végétales par utilisation d'au moins une étape de stripage par un gaz vecteur avec un gradient de température et un gradient de vide spécifiques, avantageusement à une température croissante de 80°C à 250°C et une progression de 0,5 à 2°C/min et sous un vide décroissant de 50 à 1 mbar et une progression de 0,1 à 10 mbar/min, et encore plus avantageusement à une température croissante de 145°C à 210°C et une progression de 1°C/min et sous un vide décroissant de 30 à 2 mbar et une progression de 0,5 à 5 mbar/min.

Le procédé selon l'invention, incluant notamment une telle étape de stripage, conduit à la préparation d'un insaponifiable total débarrassé substantiellement de tout composé présentant un risque de toxicité, tel que les composés sapides et/ou odorants et/ou les composés chimiques résultant de l'altération et de la dégradation des huiles végétales.

Le procédé selon l'invention comprend avantageusement la combinaison de différentes opérations qui permettent de maîtriser et de contrôler précisément l'évolution et la transformation des différents constituants spécifiques de l'insaponifiable. Le procédé selon la présente invention permet de se débarrasser substantiellement des impuretés spécifiques des co-produits de l'industrie du raffinage des huiles végétales, tels que les DD, et de garantir la qualité et l'innocuité de l'insaponifiable obtenu.

Par ailleurs, le procédé selon la présente invention permet l'obtention, avec un rendement élevé, d'un insaponifiable total purifié qui peut avantageusement être incorporé au sein de compositions cosmétiques, dermatologiques, pharmaceutiques ou de dispositifs médicaux, ou encore dans des compositions alimentaires, des compléments alimentaires ou des nutraceutiques, à visée humaine ou animale.

La présente invention a ainsi pour objet l'utilisation d'au moins un co-produit de l'industrie du raffinage des huiles végétales pour obtenir un insaponifiable total purifié d'huile végétale débarrassé des impuretés initialement présentes dans ledit co-produit, telle que définie dans la revendication 1.

L'insaponifiable d'un corps gras comprend l'ensemble des constituants qui après hydrolyse basique, telle que la saponification, sont très peu solubles ou insolubles dans l'eau et solubles dans des solvants organiques, tels que l'éther éthylique, les hydrocarbures aromatiques, les solvants chlorés....

L'insaponifiable est donc composé de tous les constituants non hydrolysables du corps gras ainsi que ceux résultant majoritairement de la saponification d'esters non glycéridiques d'acides gras (esters de stérols, cires, esters de tocophérols...). Comme mentionné ci-dessus, les quatre grandes familles qui constituent les insaponifiables sont les stérols, les tocophérols, les alcools aliphatiques et les hydrocarbures aliphatiques.

Par l'expression " insaponifiable total d'huile végétale ", on entend au sens de la présente invention tous les constituants et familles de constituants naturellement présents dans l'insaponifiable de l'huile considérée.

L'appellation insaponifiable total d'huile végétale est ainsi définie par sa composition et son mode de préparation. L'insaponifiable total renferme toutes les familles de constituants naturellement présents dans l'insaponifiable de l'huile d'origine considérée. Il est obtenu par extraction à l'aide d'un solvant organique tel que décrit dans la définition ci-dessus.

Au sens de la présente invention, l'insaponifiable total d'huile végétale n'est pas la résultante d'une ou plusieurs opérations de fractionnement ou de mélange visant à obtenir, sous forme de fraction isolée et purifiée, un composé ou une famille de composés constituant de l'insaponifiable. En outre, la composition de l'insaponifiable total d'huile végétale selon la présente invention est équivalente, en terme de pourcentages relatifs des constituants des différentes familles, à celle de l'insaponifiable de l'huile d'origine.

Par l'expression " insaponifiable total d'huile végétale purifié ", on entend au sens de la présente invention un insaponifiable total, dont les impuretés et produits toxiques notamment présents dans le produit de départ ont été éliminés, avantageusement dont la totalité ou la quasi-totalité des impuretés ou produits toxiques notamment présents dans le produit de départ ont été éliminés. L'insaponifiable total purifié d'huile végétale est obtenu par au moins une étape d'extraction de l'insaponifiable total brut d'huile végétale à partir d'au moins un co-produit de l'industrie du raffinage des huiles, typiquement par saponification et extraction liquide/liquide, puis par une étape de purification de l'insaponifiable total brut afin d'éliminer les impuretés précédemment décrites, avantageusement par stripage.

Par l'expression " insaponifiable total brut d'huile végétale ", on entend au sens de la présente invention l'insaponifiable isolé du co-produit de l'industrie du raffinage des huiles végétales et débarrassé de tous les constituants hydrolysables du corps gras, c'est-à-dire débarrassé des constituants glycérides du type acides gras, mono, di et triglycérides, préférentiellement par saponification puis extraction par un solvant organique.

Par ailleurs, avantageusement selon la présente invention, l'insaponifiable total d'huile végétale purifié est débarrassé des solvants résiduels utilisés lors de l'extraction de l'insaponifiable à partir du co-produit de l'industrie du raffinage des huiles.

En particulier, le co-produit de l'industrie du raffinage des huiles végétales utilisé dans le cadre de la présente invention est un distillat de désodorisation (DD) d'huiles végétales et/ou un condensat de raffinage physique.

De manière particulièrement avantageuse, l'insaponifiable total purifié selon l'invention est débarrassé des composés sapides et/ou odorants tels que le deca-2 ,4-diénal-(E,E), l'hexanal, l'hexanol, le 1 hexanol-2-ethyl, les terpènes comme le β-Caryophyllene..., et/ou des composés chimiques résultant de l'altération et de la dégradation des huiles végétales, tels que les aldéhydes, cétones, hydrocarbures légers et terpènes....

Un des phénomènes fondamentaux de l'altération et la dégradation des huiles est représenté par l'oxydation. L'altération chimique des corps gras insaturés par l'oxygène de l'air débute par la formation de peroxydes et ensuite par la formation de produits de scission. Ces produits de scission résultent de la coupure de la chaîne grasse au niveau de la ou des doubles liaisons qui conduit à la formation de composés à chaînes courtes comme des hydrocarbures, des aldéhydes et des cétones, tous volatils, responsables de l'odeur rance des corps gras oxydés. Lors du procédé de raffinage des corps gras, ces composés sont éliminés au cours de l'opération de désodorisation et se retrouvent concentrés dans les distillats de désodorisation. Ils se retrouvent majoritairement dans l'insaponifiable extrait et doivent être séparés sélectivement pour débarrasser l'insaponifiable total de ces composés délétères.

De par leur instabilité thermique et chimique, leur odeur et leur goût, ils confèrent aux mélanges qui les contiennent une forte instabilité organoleptique, une odeur et un goût incompatibles avec une ingestion *per os.* Ils présentent également de par leur structure chimique un potentiel de toxicité non acceptable en termes de santé publique et de sécurité.

La multitude de molécules chimiques représentée par ces composés sapides et/ou odorants et/ou les composés chimiques résultant de l'altération et la dégradation des huiles rend très difficile leur séparation du mélange insaponifiable total. La Demanderesse a ainsi découvert de manière surprenante que seule une maîtrise parfaite des paramètres opératoires que sont le vide, la température et l'injection de vapeur permet une élimination sélective et complète de ces composés sans perte significative des composés constitutifs de l'insaponifiable et sans modifications de leurs proportions relatives.

Typiquement selon la présente invention, l'insaponifiable total purifié contient au plus 1000 ppm, avantageusement au plus 500 ppm, encore plus avantageusement au plus 100 ppm, de composés sapides et/ou odorants et/ou des composés chimiques résultant de l'altération et de la dégradation des huiles végétales, par rapport à la masse totale de l'insaponifiable. Selon l'invention, les composés sapides et/ou odorants et/ ou les composés chimiques résultant de l'altération et de la dégradation des huiles végétales sont éliminés par au moins une étape d'entraînement par stripage par un gaz vecteur avec un gradient de température et de vide, avantageusement à une température croissante de 80°C à 250°C, typiquement de 145°C à 210°C, et une progression de 0,5 à 2°C/min, typiquement de 1°C/min, et sous un vide décroissant de 50 à 1 mbar, typiquement de 30 à 2 mbar, et une progression de 0,1 à 10 mbar/min, typiquement de 0,5 à 5 mbar/min.

Plus particulièrement, l'étape de stripage par un gaz vecteur est réalisée avec un gradient de température et de vide, à une température croissante de 145°C à 210°C et une progression de 1°C/min, et sous un vide décroissant de 30 à 2 mbar et une progression de 0,5 à 5 mbar/min.

Par ailleurs, avantageusement selon l'invention, l'insaponifiable total purifié est débarrassé des résidus de produits phytosanitaires du type pesticides, tels que l'aldrine, la dieldrine, les endosulfans alpha, beta et sulfate ou encore le pyrimiphos-méthyl, dichlorvos et malathion, avantageusement par au moins une étape de stripage par un gaz vecteur sous vide, typiquement à une température comprise entre 180°C et 250 °C et sous un vide de 1 à 5 mbar.

Les produits phytosanitaires sont des composés utilisés sur les cultures et lors du stockage des graines ou sont présents de façon plus globale dans notre environnement. Ils peuvent ainsi se retrouver concentrés dans les produits extraits des végétaux. Il convient de les éliminer pour garantir aux produits fabriqués des teneurs résiduelles compatibles avec les spécifications proposées par les différentes règlementations (Limite Maximale de Résidu, LMR) et assurer la maîtrise de leurs risques chimiques.

De manière avantageuse selon l'invention, la quasi-totalité des résidus de pesticides présents dans la matière de départ (co-produit) est éliminée ou la teneur résiduelle dans le produit préparé (insaponifiable total purifié) est réduite en dessous de la limite quantifiable définie pour les méthodes analytiques utilisées.

Typiquement, la teneur en résidus de produits phytosanitaires, tels que les pesticides, est réduite d'au moins 95%, avantageusement d'au moins 98%, et encore plus avantageusement d'au moins 99% dans l'insaponifiable total purifié, par rapport à la teneur dans la matière de départ (co-produit de l'industrie du raffinage des huiles).

De manière particulièrement avantageuse selon l'invention, l'insaponifiable total purifié d'huile végétale est débarrassé des composés de types suivants : des composés sapides et/ou odorants, des composés chimiques résultant de l'altération et la dégradation des huiles, tels que les aldéhydes, cétones, hydrocarbures légers, des traces de résidus phytosanitaires tels que les pesticides. En outre, la teneur résiduelle en contaminants qui peuvent résulter de contaminations croisées (brassicastérol...) est avantageusement réduite.

Typiquement, dans le cadre de la présente invention, les co-produits de l'industrie du raffinage des huiles végétales sont issus d'huile de soja, de tournesol, de colza, de germe de blé, de germe de maïs, d'olive, de palme, de palmiste, de coton, de coprah, de sésame, de lupin, de noix de coco, d'arachide, de lin, de ricin, de pépins de raisin, de pépins de courge, de pépins de cassis, de pépins de melon, de pépins de tomate, de pépins de citrouille, d'amande, de noisette, de noix, d'onagre, de bourrache, de carthame, de cameline, d'oeillette.

Dans un mode de réalisation particulier selon la présente invention, l'huile végétale utilisée dans le cadre de l'invention est l'huile de soja ou l'huile de tournesol.

De manière particulièrement avantageuse, l'huile végétale utilisée dans le cadre de l'invention est l'huile de soja.

La présente invention a également pour objet un procédé d'obtention d'un insaponifiable total purifié d'huile végétale à partir d'au moins un co-produit de l'industrie du raffinage des huiles végétales, ledit insaponifiable total purifié étant débarrassé des impuretés initialement présentes dans ledit co-produit, tel que défini dans la revendication 4.

Le procédé objet de la présente invention comprend au moins une étape d'extraction de l'insaponifiable total brut d'huile végétale à partir du ou des co-produit(s) de l'industrie du raffinage des huiles, typiquement par saponification et extraction liquide/liquide, ainsi qu'une étape de purification de l'insaponifiable total brut, afin d'éliminer les impuretés précédemment décrites, avantageusement par stripage.

En particulier, le procédé objet de la présente invention comprend les étapes successives suivantes :

(1) Saponification en milieu hydro-alcoolique par une base alcaline du type potasse,

(2) Extraction liquide-liquide par un solvant organique du mélange réactionnel issu de la saponification préalablement dilué avec de l'eau,

(3) Lavage à l'eau de la solution organique extraite, avantageusement par extraction liquide/liquide,

(4) Evaporation du solvant organique, puis

(5) Stripage par un gaz vecteur sous vide afin d'obtenir un insaponifiable total purifié d'huile végétale,

caractérisé en ce que le stripage (5) par un gaz vecteur sous vide comprend au moins une étape (b) d'entraînement avec un gradient de température et de vide, avantageusement à une température croissant de 80°C à 250°C, typiquement de 145°C à 210°C, et une progression de 0,5 à 2°C/min, typiquement de 1 °C/min, et sous un vide décroissant de 50 à 1 mbar, typiquement de 30 à 2 mbar, et une progression de 0,1 à 10 mbar/min, typiquement de 0,5 à 5 mbar/min, afin d'éliminer les composés sapides et/ou odorants et/ou les composés chimiques résultant de l'altération et de la dégradation des huiles végétales.

Le procédé, objet de la présente invention, comprend avantageusement une étape préalable de concentration en insaponifiable du co-produit de l'industrie du raffinage des huiles végétales, typiquement par distillation moléculaire, avant l'étape (1) de saponification.

Il s'agit ainsi préférentiellement d'une concentration par distillation moléculaire.

Cette opération peut être intégrée au procédé pour permettre un enrichissement préalable des co-produits tels que les Distillats de Désodorisation (DD) faiblement titrés en insaponifiable. Cette étape préliminaire est basée sur l'écart des valeurs de la tension de vapeur qui existe entre les acides gras constituants majoritaires des DD faiblement titrés en insaponifiable et les composants constitutifs de l'insaponifiable.

Cette opération unitaire a pour objet d'obtenir, par distillation, une fraction enrichie en insaponifiable afin de minimiser les quantités à saponifier et à extraire lors des opérations suivantes. Par utilisation de cette étape, l'objectif n'est pas de purifier le produit, mais de retirer du DD une fraction concentrée renfermant le maximum de composés recherchés. De ce fait, la coupe effectuée n'est pas critique et sa maîtrise n'est légitimée que par le souci d'engager dans les phases aval un intermédiaire enrichi en insaponifiable.

Le procédé spécifique de distillation moléculaire, qui permet le fractionnement sans chauffage permanent de l'intégralité du milieu, est utilisé de manière à minimiser les dégradations potentielles des constituants thermosensibles et oxydables.

L'intérêt d'utiliser cette technologie à ce stade du procédé est de réduire les volumes à traiter dans les étapes amont par concentration de la matière première et ainsi réduire les quantités de réactifs mis en oeuvre, les volumes de solvants utilisés, les volumes de rejets et plus globalement augmenter la productivité des installations et abaisser les prix de revient des insaponifiables totaux d'huiles végétales.

Selon une caractéristique particulière de la présente invention, le procédé comprend ainsi une étape préalable de distillation moléculaire, de préférence à une température de 100°C à 150°C environ en maintenant une pression comprise entre $10^{-3}$ et $10^{-2}$ mmHg, soit 0,13 à 1,3 Pa, par exemple de l'ordre de $10^{-3}$ mmHg. Le co-produit est distillé typiquement dans un distillateur moléculaire de type centrifuge ou à film raclé. Cette étape de distillation moléculaire permet de concentrer d'un facteur 2 à 20 la teneur en insaponifiable des co-produits mis en oeuvre.

L'étape (1) de saponification du co-poduit de type DD du procédé est réalisée en milieu hydro-alcoolique par une base alcaline du type potasse.

La saponification des corps gras végétaux par une base alcaline est une réaction classiquement utilisée dans le domaine de huilerie et de la savonnerie, et encore plus particulièrement dans les méthodes analytiques visant à doser l'insaponifiable des corps gras. Par la mise en oeuvre de cette réaction chimique, le but recherché est de parvenir à une réaction complète caractérisée par une absence d'esters résiduels. Les moyens mis en oeuvre pour y parvenir sont systématiquement l'utilisation d'un excès important de réactif et un milieu réactionnel homogène. L'utilisation d'un alcool comme solvant réactionnel permet de faciliter le contact entre les réactifs et de maintenir un reflux propice à un temps de réaction optimum.

Avantageusement, la base utilisée est de la potasse ou de la soude. Typiquement, le milieu hydro-alcoolique est un mélange d'eau et d'alcool à chaînes courtes du type méthanol ou éthanol principalement.

L'étape (2) d'extraction par un solvant organique est avantageusement réalisée sur colonne d'extraction liquide/liquide.

Cette étape permet d'extraire les constituants de l'insaponifiable au moyen d'un solvant organique approprié. Elle est avantageusement précédée d'une opération de dilution par de l'eau du milieu réactionnel pour ajuster le titre en alcool de la solution et l'adapter à l'équilibre du système obtenu avec le solvant organique. A l'aide d'une colonne d'extraction liquide/liquide, l'injection du solvant à contre-courant de la solution hydro-alcoolique permet la séparation des composés hydrophiles (savons, glycérol, etc.) des composés hydrophobes (insaponifiable) par solubilisation sélective de ces derniers dans le solvant organique.

Différents solvants organiques peuvent être utilisés lors de l'extraction liquide-liquide (2), tels que les alcanes, les solvants chlorés, les solvants aromatiques fluorés, les tert-butyl éthers, les solvants comprenant un atome de silicium, le MeTHF, et leurs mélanges.

Dans un exemple de réalisation particulier selon la présente invention, le solvant organique de l'extraction liquide/liquide (2) est le 1,2-dichloroéthane (DCE).

L'étape (3) de lavage à l'eau de la solution organique extraite est une étape de purification avantageusement réalisée par extraction liquide/liquide, typiquement sur colonne extraction liquide/liquide (L/L).

La démixtion entre les deux phases en contact lors de l'extraction n'est pas totale et des fines gouttelettes de solution hydro-alcoolique restent en dispersion dans la solution organique contenant l'insaponifiable en solution. Ces gouttelettes renferment des composés hydrophiles tels que les savons, le glycérol, l'alcool... qu'il convient d'éliminer.

En général réalisée en mode continu, l'opération de lavage fait appel à une colonne permettant l'injection de l'eau de lavage à contre-courant de la phase organique. Le rapport de volume phase organique/eau de lavage est ajusté pour obtenir une efficacité maximum avec un minimum de consommation en eau. Les proportions classiquement utilisées dépendent du type de colonne d'extraction utilisé et varient de 0,25 à 4, exprimées en rapport de volume d'eau de lavage par rapport au volume de solution organique à laver.

L'étape (4) d'évaporation du solvant organique consiste à éliminer le solvant organique avant de procéder à l'étape (5) de stripage.

Elle est réalisée sur tout matériel classiquement utilisé pour évaporer un solvant et récupérer un résidu partiellement ou totalement désolvanté. A titre d'exemple, un évaporateur à film tombant ou un évaporateur à plaques peut être utilisé pour cette opération.

A ce stade du procédé, le produit récupéré n'est généralement pas complètement débarrassé du solvant organique, car les technologies utilisées et les conditions opératoires (température, pression) ne permettent pas d'éliminer la totalité du solvant. Une opération complémentaire est ainsi généralement nécessaire pour finaliser la séparation du solvant et garantir une teneur résiduelle en solvant compatible avec une utilisation cosmétique, nutraceutique ou pharmaceutique ou comme dispositifs médicaux.

Il est donc important d'éliminer tous les solvants résiduels dans la mesure du possible, de façon à se conformer aux spécifications du produit, aux bonnes pratiques de fabrication ou à d'autres exigences liées à la qualité. Les produits finis ne peuvent donc pas contenir des concentrations de solvants résiduels dépassant les niveaux d'innocuité. Certains solvants sont en effet connus pour leur toxicité.

Avantageusement selon l'invention, le stripage par un gaz vecteur sous vide (5) comprend une étape préliminaire (a) d'élimination du solvant organique résiduel, avantageusement à une température comprise entre 80°C et 145 °C et sous un vide de 200 à 30 mbar. Selon l'invention, le stripage (5) par un gaz vecteur sous vide comprend au moins une étape (b) d'entraînement des composés sapides et/ou odorants et/ou les composés chimiques résultant de l'altération et de la dégradation des huiles végétales, en utilisant un gradient de température et de vide spécifique. Typiquement, cette étape (b) est réalisée suite à l'étape (a) mentionnée ci-dessus.

L'étape (b) du stripage (5) par un gaz vecteur sous vide est avantageusement réalisée à une température croissante de 80°C à 250°C, typiquement de 145°C à 210°C, et une progression de 0,5 à 2°C/min, typiquement de 1°C/min, et sous un vide décroissant de 50 à 1 mbar, typiquement de 30 à 2 mbar, et une progression de 0,1 à 10 mbar/min, typiquement de 0,5 à 5 mbar/min, afin d'éliminer les composés sapides et/ou odorants et/ou les composés chimiques résultant de l'altération et de la dégradation des huiles végétales.

Plus particulièrement, l'étape (b) de stripage (5) par un gaz vecteur est réalisée à une température croissante de 145°C à 210°C et une progression de 1°C/min, et sous un vide décroissant de 30 à 2 mbar et une progression de 0,5 à 5 mbar/min.

Un objectif complémentaire de cette étape de stripage (5) est ainsi représenté par l'élimination systématique des composés sapides et/ou odorants et/ou des composés chimiques résultant de l'altération et la dégradation des huiles (aldéhydes, cétones, hydrocarbures légers,...), et avantageusement des traces de résidus phytosanitaires.

De manière particulièrement avantageuse selon l'invention, le stripage (5) par un gaz vecteur sous vide comprend en outre une étape (c) d'élimination des résidus de produits phytosanitaires du type pesticides, avantageusement à une température comprise entre 180°C et 250 °C et sous un vide de 1 à 5 mbar, et encore plus avantageusement à une température de 210°C sous un vide de 2 mbar. Typiquement, cette étape (c) est réalisée suite à l'étape (b) mentionnée ci-dessus.

L'étape ultime de stripage vapeur selon l'invention correspond à l'élimination des résidus de produits phytosanitaires tels que les pesticides.

Comme vu ci-dessus, il convient de les éliminer pour garantir aux produits fabriqués des teneurs résiduelles compatibles avec les spécifications proposées par les différentes règlementations (Limite Maximale de Résidu, LMR) et assurer la maîtrise du risque chimique. L'étape (c) permet ainsi avantageusement de préparer un insaponifiable total purifié d'huile végétale contenant au plus une teneur égale à la LMR (Limite Maximale de Résidu) fixée par la directive européenne en vigueur pour les pesticides.

Il a ainsi été découvert que l'étape de stripage gazeux selon l'invention en appliquant des conditions particulières de température, de vide et de débit de vapeur sèche ou gaz vecteur permet une élimination sélective des impuretés sans perte des constituants majeurs de l'insaponifiable et sans modification de leurs proportions relatives. Les conditions particulières sont obtenues en appliquant un gradient de température corrélé au vide, c'est-à-dire en faisant progresser parallèlement la consigne de température et de vide afin d'obtenir une distillation sélective des constituants du mélange.

Ceci est parfaitement atteint par le procédé selon l'invention par une programmation judicieuse des gradients de température et du vide respectant des phases de progression linéaire entrecoupées de phases stabilisées lors de l'étape de stripage (5).

C'est par la définition d'une phase précise du procédé et un contrôle permanent des paramètres de vide et de température et d'ajout du gaz vecteur qu'une efficacité maximum de l'élimination totale des différents constituants peut être obtenue. Généralement, l'élimination totale des composés sapides et/ou odorants et/ou des composés chimiques résultant de l'altération et de la dégradation des huiles végétales, ne peut être obtenue que si la totalité du solvant est éliminée et de même pour les pesticides si les deux phases précédentes ont été parfaitement maîtrisées.

Selon une caractéristique particulière selon l'invention, le gaz vecteur du stripage (5) est la vapeur sèche ou un gaz neutre comme l'azote.

De manière particulièrement avantageuse selon l'invention, l'ensemble des opérations de stripage (5) par un gaz vecteur sous vide, en particulier les étapes (a), (b) et (c) de stripage sont réalisées dans un seul et même appareil, en faisant varier successivement les différents paramètres opératoires qui permettent l'élimination progressive et maîtrisée des impuretés présentées.

Ceci conduit ainsi à la réduction du nombre d'étapes du procédé et à l'amélioration de son rendement et de sa productivité.

Le rassemblement de ces différentes phases du stripage dans un même équipement permet avantageusement de réduire ainsi les temps de cycle, les besoins en énergie par un enchaînement des phases sans transfert ou refroidissement et de limiter le temps de maintien à haute température, réduisant ainsi les risques de décomposition thermique et oxydative.

De façon inattendue, la présente invention conduit également à l'abaissement de la teneur relative en contaminants, tels que le brassicastérol qui peuvent résulter de contaminations croisées. Un avantage significatif de cette propriété est de pouvoir corriger partiellement les impacts liés à l'utilisation de la matière première DD et à ses risques inhérents de contaminations croisées entre différentes sources d'huiles végétales, dont la présence d'huile de colza. Dans le cas spécifique de l'insaponifiable total d'huile de soja, le procédé selon l'invention, et en particulier les étapes de stripage (5), notamment l'étape (b), conduit avantageusement à un abaissement en dessous de la teneur relative de 0,3% nécessaire pour répondre aux exigences de la réglementation, en particulier lorsque l'huile végétale est l'huile de soja.

L'invention conduit également de façon très avantageuse à un insaponifiable total d'huile végétale présentant toutes les caractéristiques de composition de la matière première dont il est issu et, plus particulièrement, de l'huile végétale d'origine. Cette propriété est obtenue grâce à l'élimination sélective des différentes impuretés sans pertes des composés d'intérêts.

Il est ci-dessous décrit un insaponifiable total purifié d'huile végétale susceptible d'être obtenu par le procédé selon l'invention, ou directement obtenu par le procédé selon l'invention, contenant au plus 1000 ppm, avantageusement au plus 500 ppm, encore plus avantageusement au plus 100 ppm, de composés sapides et/ou odorants et/ou des composés chimiques résultant de l'altération et de la dégradation des huiles végétales, par rapport à la masse totale de l'insaponifiable.

Avantageusement, un tel insaponifiable a une teneur résiduelle en produits phytosanitaires, tels que les pesticides, d'au plus 5 %, avantageusement d'au plus 2 %, et encore plus avantageusement d'au plus 1 %, par rapport à la teneur dans le produit de départ du type co-produit de l'industrie du raffinage des huiles végétales.

Typiquement, la teneur résiduelle en produits phytosanitaires tels que les pesticides est ainsi réduite dans l'insaponifiable total purifié d'au moins 95%, avantageusement d'au moins 98%, et encore plus avantageusement d'au moins 99%, par rapport à la teneur dans la matière de départ (co-produit de l'industrie du raffinage des huiles).

Selon une caractéristique particulière l'insaponifiable total purifié d'huile végétale contient au plus 100 ppm, avantageusement au plus 10 ppm, encore plus avantageusement au plus 5 ppm, de solvant résiduel, par rapport à la masse totale de l'insaponifiable.

Selon un exemple particulier l'insaponifiable total purifié d'huile végétale est un insaponifiable total purifié d'huile de soja et ledit insaponifiable total purifié d'huile de soja contient une teneur en brassicastérol inférieure ou égale à 0,3% par rapport à la masse des stérols totaux de l'insaponifiable.

Typiquement, l'insaponifiable total purifié d'huile végétale est un insaponifiable total purifié d'huile de soja qui contient du squalène à une teneur comprise entre 0,5 et 15%, avantageusement entre 1 et 10% en masse, par rapport à la masse totale de l'insaponifiable.

Typiquement, un tel insaponifiable total purifié d'huile de soja contient des tocophérols à une teneur comprise entre 10 et 50%, avantageusement entre 20 et 40% en masse, par rapport à la masse totale de l'insaponifiable.

Avantageusement, lesdits tocophérols contiennent de l'alpha-tocophérol à une teneur comprise entre 2 et 40%, avantageusement entre 5 et 20% en masse, par rapport à la masse totale des tocophérols, du gamma-tocophérol à une teneur comprise entre 40 et 80%, avantageusement entre 50 et 70% en masse, par rapport à la masse totale des tocophérols, et du delta-tocophérol à une teneur comprise entre 10 et 50%, avantageusement entre 15 et 40% en masse, par rapport à la masse totale des tocophérols, dans l'insaponifiable total purifié d'huile de soja.

Typiquement, un tel insaponifiable total purifié d'huile de soja contient également des stérols à une teneur comprise entre 30 et 70%, avantageusement entre 35 et 65% en masse, par rapport à la masse totale de l'insaponifiable.

Avantageusement, lesdits stérols contiennent du campestérol à une teneur comprise entre 10 et 40%, avantageusement entre 15 et 30% en masse, par rapport à la masse totale des stérols, du stigmastérol à une teneur comprise entre 10 et 35%, avantageusement entre 15 et 25% en masse, par rapport à la masse totale des stérols, et du beta-sitostérol à une teneur comprise entre 30 et 60%, avantageusement entre 35 et 50% en masse, par rapport à la masse totale des stérols, dans l'insaponifiable total purifié d'huile de soja.

De manière particulièrement avantageuse l'insaponifiable total purifié d'huile végétale est un insaponifiable total purifié d'huile de soja contenant du squalène, des tocophérols, en particulier de l'alpha tocophérol, du gamma tocophérol et du delta tocophérol, ainsi que des stérols, en particulier du campestérol, du stigmastérol et du beta-sitostérol, aux teneurs mentionnées ci-dessus.

Il est également décrit une composition contenant un insaponifiable total purifié d'huile végétale, avantageusement à une concentration comprise entre 0,1 et 98% en masse, encore plus avantageusement de 30 à 70% en masse, par rapport à la masse totale de la composition.

En particulier, la composition contient un insaponifiable total purifié d'huile de soja tel que décrit ci-dessus en association avec un insaponifiable d'avocat tel qu'un insaponifiable d'avocat furanique ou un insaponifiable d'avocat stérolique, de préférence avec un insaponifiable d'avocat furanique, avantageusement dans un rapport d'environ 2/3 pour le soja et 1/3 pour l'avocat.

Enfin, il est également décrit un insaponifiable total purifié d'huile végétale tel que décrit ci-dessus ou la composition telle que décrite ci-dessus pour son utilisation comme médicament, comme dispositif médical, comme agent dermatologique, comme agent cosmétique, ou comme nutraceutique, à visée humaine ou animale, avantageusement dans la prévention et/ou le traitement des troubles du tissu conjonctif, tels que l'arthrose, des pathologies articulaires telles que les rhumatismes, des maladies parodontales, telles que la gingivite ou la parodontite, ou encore dans la prévention et/ou le traitement des troubles du derme et/ou de l'hypoderme tels que le vieillissement cutané, les vergetures et la cellulite, ou encore des troubles de la barrière épidermique tels que les inflammations cutanées, les peaux à tendance atopique, l'eczéma atopique et les dermatites irritatives et/ou inflammatoires.

En particulier, le procédé selon l'invention conduit à la préparation d'un insaponifiable total d'huile végétale, et dans le cas spécifique d'une matière première comme le soja à un produit utilisable, en association avec un insaponifiable d'huile d'avocat, dans la fabrication de médicaments destinés au traitement de l'arthrose.

De par sa composition particulière liée à la présence de tous les constituants de l'insaponifiable total d'huile de soja, un tel insaponifiable total purifié d'huile de soja a montré une activité biologique supérieure à ses fractions purifiées, notamment les stérols, sur les différents aspects du métabolisme des chondrocytes, fibroblastes, odontoblastes.

Dans un exemple de réalisation particulier, l'insaponifiable total purifié d'huile végétale de soja peut être utilisé en association avec un insaponifiable d'avocat, avantageusement dans un mélange d'insaponifiable d'avocat furanique et d'insaponifiable de soja, dans un rapport respectif d'environ 1/3-2/3.

Par ailleurs, on entend par dispositif médical tout instrument, appareil, équipement, matière, produit, à l'exception des produits d'origine humaine, ou autre article, seul ou en association, destiné par le fabricant à être utilisé chez l'homme à des fins médicales, et dont l'action principale voulue n'est pas obtenue par des moyens pharmacologiques ou immunologiques ni par métabolisme, mais dont la fonction peut être assistée par de tels moyens.

Les crèmes ou autres produits topiques peuvent également être des dispositifs médicaux, comme c'est le cas par exemple pour la prise en charge de la dermatite atopique.

Enfin, il est décrit l'utilisation cosmétique d'un insaponifiable total purifié d'huile végétale tel que décrit ci-dessus ou d'une composition telle que décrite ci-dessus pour la prévention et/ou le traitement des troubles cutanés du derme et/ou de l'hypoderme, tels que le vieillissement, les vergetures et la cellulite ou encore pour la prévention et/ou le traitement des troubles de la barrière épidermique, tels que les peaux à tendance atopique et les peaux présentant des dermatites irritatives et/ou inflammatoires.

Les exemples suivants sont donnés pour illustrer l'invention :

Exemple 1 :

Composition de distillat de désodorisation de soja

Le marché des huiles, et plus particulièrement de l'huile de soja et des DD, est fortement conditionné par la disponibilité des graines au niveau mondial et par les besoins sans cesse croissants en vitamine E et phytostérols pour des développements sur des applications alimentaires, cosmétiques ou pharmaceutiques.

Cette demande incite les industriels des huileries à optimiser les procédés de désodorisation pour aboutir à des DD possédant une composition très favorable pour la valorisation des fractions insaponifiables.

Les modifications apportées sur les appareils de désodorisation visent principalement à augmenter la température de désodorisation et à installer des condenseurs spécifiques qui permettent de recueillir des distillats de désodorisation particulièrement riches en tocophérols et phytostérols.

C'est cette qualité de DD qui sera traitée préférentiellement par la nouvelle invention.

A titre d'exemple, une composition de distillat de désodorisation de soja est donnée :

| | |
|---|---|
| Stérols et alcools triterpéniques | 21 % |
| Tocophérols | 18 % |
| Squalène | 4 % |
| Composés sapides et odorants | 7 % |
| Acides gras | 23 % |
| Glycérides | 27 % |

Exemple 2 :

Distillation d'un distillat de désodorisation de soja

Le procédé objet de la présente invention peut comprendre avantageusement un traitement préliminaire par distillation moléculaire dans le cas de DD à teneur réduite en insaponifiable. Il sera appliqué pour éliminer une partie de la fraction légère des DD, constituée essentiellement d'acides gras, afin d'obtenir ainsi un enrichissement en insaponifiable de la matière première engagée dans le procédé.

Cet essai a été réalisé sur un distillateur moléculaire pilote de type centrifuge, d'une capacité maximale de 25 kg/heure. Cet appareil est constitué des éléments suivants :

Un rotor circulaire conique de 38 cm de diamètre,
Un serpentin à circulation d'eau (condenseur),
Deux gouttières de récupération du résidu et du distillat,
Un système de chauffage par induction.

L'unité de vide est quant à elle composée d'une pompe à palette (vide primaire) et d'une pompe à diffusion d'huile (vide moléculaire).

Le produit à traiter est préalablement pompé vers un dégazeur continu, constitué d'un évaporateur de type couche mince, à film tombant. Ce prétraitement permet l'élimination avant distillation, des éventuelles traces d'eau (ou de solvant) et de gaz dissous que pourrait contenir la matière première. Le produit ainsi dégazé est ensuite repris par une nouvelle pompe, puis transféré dans l'enceinte de distillation (cloche sous vide moléculaire). L'alimentation en liquide se fait au centre du disque tournant. Ainsi, sous l'effet de la force centrifuge, le produit à distiller s'étale sur le rotor sous la forme d'un film mince. Les vapeurs formées au cours du processus de distillation seront alors condensées sur les parois de la cloche et sur le serpentin à circulation d'eau prévu à cet effet. Le distillat obtenu, à l'instar de la fraction lourde s'écoulant par une gouttière adjacente, est repris par une pompe et ainsi récupéré.

Les paramètres utilisés pour cette opération sont les suivants :

Débit alimentation : 16 kg/h
Vide : $10^{-2}$ mm de Hg
Température de distillation : 110°C
Taux de distillation : 44,5 %

Composition des produits :

| Composés en g pour 100 g | Matière première DD | Résidu distillation |
|---|---|---|
| Stérols et alcools triterpéniques | 22,0 | 36,5 |
| Tocophérols | 10,1 | 17,1 |
| Squalène | 6,1 | 6,9 |
| Composés sapides et odorants | 21,8 | 6,0 |
| Acides gras et glycérides | 27 | 31 |

Exemple 3 :

Distillation d'un distillat de désodorisation de tournesol

Cet essai a été conduit sur le même appareillage que l'exemple n°2. Les paramètres utilisés pour cette opération sont les suivants :

Débit alimentation : 18,5 kg/h
Vide : $10^{-2}$ mm de Hg
Température de distillation : 100°C
Taux de distillation : 82,5 %

Composition des produits :

| Composés en g pour 100 g | Matière première DD | Résidu distillation |
|---|---|---|
| Stérols et alcools triterpéniques | 1,1 | 7,9 |
| Tocophérols | 0,1 | 0,6 |
| Squalène | 0,4 | 1,7 |
| Composés sapides et odorants | 82 | 53,6 |
| Insaponifiable total | 7,8 | 18,5 |
| Acides gras et glycérides | 10,2 | 31 |

Exemple 4 :

Mise en oeuvre du procédé sur le soja

Matière première mise en oeuvre :
Distillat de désodorisation d'huile de soja
Composition :

Teneur en insaponifiable total : 35,8 %
Teneur en Stérols : 20,5 %
Teneur en tocophérols : 12,8 %
Teneur en squalène : 2,6 %
Teneur en composés sapides et odorants : 3,5%

Composition relative en stérols

Beta-sitostérol : 42,3 %
Campestérol : 21,4 %
Stigmastérol : 20,9 %
Brassicastérol : 0,2 %

Composition relative en tocophérols

Alpha-tocophérol : 15,3 %
Delta-tocophérol : 28,3 %
Gamma-tocophérol : 56,4 %

Etape (1) Saponification :

Dans un réacteur de 50L muni d'une agitation mécanique type ancre, d'une double enveloppe chauffage vapeur et d'un condenseur de reflux, sont introduits successivement :

8,84 Kg de distillat de désodorisation de soja
2,7 Kg de solution de potasse
20,6 L d'alcool

Le mélange est porté à reflux sous agitation pendant 3h.
Après dilution avec de l'eau, le mélange réactionnel est transféré dans une cuve inerte à l'azote qui servira à l'alimentation de la colonne d'extraction liquide/liquide.

Etape (2) Extraction :

Colonne diamètre 60 mm
Débit solution à extraire → 15L/h
Solvant d'extraction : 1,2-Dichloroéthane
Débit solvant d'extraction → 18L/h

Etape (3) Lavage :

Colonne diamètre 60 mm
Débit solution à laver → 20 L/h
Ratio Eau/solution à lavée : 1
Débit d'eau → 20 L/h

Etape (4) Purification par évaporation :

La solution lavée est évaporée par traitement sur un évaporateur du type flot tombant tubulaire chauffée par de la vapeur à 3 bars.
La solution est alimentée avec un débit régulier de 6L/H et la température de recirculation est régulée à 90°C ;
Lorsque toute la solution a été transférée, la température est montée progressivement à 115°C et l'évaporation est arrêtée dès qu'un débit de solvant distillé n'est plus détecté.

Etape (5) Purification par stripage (stripping) sous vide :

Le produit est alors transféré dans le réacteur de stripage et l'opération est enchaînée.
Après chargement du produit dans la cuve, la température est régulée à 90 °C, puis le vide est descendu progressivement à 50 mbar en 1 heure.
La température est alors montée à 145°C avec une incrémentation de 1°C/min.
Dès que la température est atteinte et qu'un débit de solvant n'est plus détecté en sortie du condenseur, de la vapeur sèche est injectée par le sparger avec un débit contrôlé pour maintenir un bouillonnement maîtrisé.
Après 1 h d'injection, la température est montée à 210°C avec une incrémentation de 1°C/min et conjointement le vide est descendu à 2 mbar avec une progression de 1 mbar/min.

Le distillat représenté par les composés sapides et/ou odorants et/ou les composés chimiques résultant de l'altération et de la dégradation de l'huile est condensé est refroidi par le condenseur, recueilli dans le pot de recette, puis évacué vers l'extérieur par une pompe.

L'addition de vapeur est alors maintenue pendant 5 h à la température de 210°C. Le produit est alors refroidi sous courant d'azote, puis soutiré après cassage du vide.

Le bilan massique de l'opération est le suivant :

Distillat de désodorisation mis en oeuvre : 8,84 kg

Composés sapides et odorants récupérés : 0,55 kg

Insaponifiable total de soja préparé : 2,98 kg

Résultats :

| | | | DD | IT | % récupérés |
|---|---|---|---|---|---|
| Teneur en Stérols (CPG) | | | 18,5% | 53,2% | 96,9 |
| Relatif | | Brassicastérol | 0,2% | 0,1% | 16,8 |
| | Relatif | Campestérol | 21,4% | 20,5% | |
| | | Stigmastérol | 20,9% | 20,6% | |
| | | Béta sitostérol | 42,3% | 42,1% | |
| Teneur en Tocophérols totaux (HPLC) | | | 12,8% | 35,4% | 93,1 |
| | Relatif | Alpha-tocophérol | 15,3% | 15,1% | |
| | | Gamma tocophérol | 56,4% | 61,6% | |
| | | Delta tocophérol | 28,3% | 23,3% | |
| Teneur en squalène | | | 2,6% | 4,1% | 53,1 |
| Teneur en composés sapides et odorants | | | 3,5% | 0,01% | |
| Résidus de pesticides mg/kg | | | 103[1] | < LOQ[2] | < 99,5[3] |
| Teneur en insaponifiable | | | 35,8%[4] | 33,7%[5] | 94,1[6] |

1- Somme des endosulfans alpha, beta et sulfate.
2- Limite quantifiable : 0,5 mg/kg pour la somme des endosulfans.
3- % de résidus de pesticides éliminés par rapport à la teneur dans le produit de départ : (103 - 0,5) x 100 / 103 = 99,5 %.
4- Teneur en insaponifiable dosée dans le distillat de désodorisation.
5- % Rendement en insaponifiable : masse d'insaponifiable total purifié obtenue par rapport à la masse de distillat de désodorisation mise en oeuvre.
6- % Rendement d'extraction : masse d'insaponifiable total purifié obtenue par rapport à la masse théorique dosée dans le distillat de désodorisation mis en oeuvre.

La composition de l'insaponifiable total de soja purifié préparé met en évidence les performances du procédé revendiqué, à savoir :

- préparer un insaponifiable total d'huile de soja purifié à partir d'un distillat de désodorisation de soja,
- garantir la présence des différentes familles de composants constitutives d'un insaponifiable de soja,
- garantir la conservation de la composition relative des différentes familles de composants,
- éliminer les composés sapides et/ou odorants et/ou les composés chimiques résultant de l'altération et de la dégradation des huiles végétales,
- réduire la teneur relative en brassicastérol,
- éliminer les traces de résidus de pesticides,
- garantir un taux de récupération important des différents composants, et notamment des stérols et des tocophérols.

Exemple 5 :

Mise en oeuvre du procédé sur le tournesol

Un insaponifiable total de tournesol est préparé à partir de la matière première distillée dans l'exemple 2 et en utilisant la technologie décrite dans l'exemple 4.

Les paramètres particuliers utilisés sont les suivants :

Matière première mise en oeuvre :

Distillat de désodorisation d'huile de tournesol

Composition :

Teneur en insaponifiable total : 18,5 %
Teneur en Stérols : 7,9 %
Teneur en tocophérols : 0,5 %
Teneur en squalène : 1,7 %
Teneur en composés sapides et odorants : 53,6 %

Composition relative en stérols

Beta-sitostérol : 56,9 %
Campestérol : 11,1 %
Stigmastérol : 12,4 %
Delta7-stigmastérol: 5 %
Brassicastérol : 0,0 %

Composition relative en tocophérols

Alpha-tocophérol : 87,5 %
Beta-tocophérol : 8,8 %
Gamma-tocophérol : 3,0 %

Etape (1) Saponification :

Dans un réacteur de 50L muni d'une agitation mécanique type ancre, d'une double enveloppe chauffage vapeur et d'un condenseur de reflux, sont introduits successivement :

18 Kg de distillat de désodorisation de tournesol concentré par distillation moléculaire
9,5 Kg de solution de potasse
40 L d'alcool

Le mélange est porté à reflux sous agitation pendant 5 h.

Après dilution avec de l'eau, le mélange réactionnel est transféré dans une cuve inertée à l'azote qui servira à l'alimentation de la colonne d'extraction liquide/liquide agitée.

Etape (2) Extraction :

Diamètre de la colonne : 60 mm
Débit solution à extraire → 15L/h
Solvant d'extraction : 1,2-Dichloroéthane
Débit solvant d'extraction → 22,5 L/h

Etape (3) Lavage :

Colonne diamètre 60 mm
Débit solution à laver → 15 L/h
Ratio Eau/solution à lavée : 1

Débit d'eau → 15 L/h

Etape (4) Purification par évaporation :

La solution lavée est évaporée par traitement sur un évaporateur du type flot tombant tubulaire chauffé par de la vapeur à 3 bars.
La solution est alimentée avec un débit régulier de 6L/H et la température de recirculation est régulée à 90°C ;
Lorsque toute la solution a été transférée, la température est montée progressivement à 115°C et l'évaporation est arrêtée dès qu'un débit de solvant distillé n'est plus détecté.

Etape (5) Purification par stripage vapeur (stripping) sous vide :

Le produit est alors transféré dans le réacteur de stripage et l'opération est enchaînée.
Après chargement du produit dans la cuve, la température est régulée à 80 °C, puis le vide est descendu progressivement à 50 mbar en 1 heure.
La température est alors montée à 125°C avec une incrémentation de 0,5°C/min.
Dès que la température est atteinte et qu'un débit de solvant n'est plus détecté en sortie du condenseur, de la vapeur sèche est injectée par le sparger avec un débit contrôlé pour maintenir un bouillonnement maîtrisé.
Après 1 h d'injection, la température est montée à 210°C avec une incrémentation de 1°C/ min et dans le même temps le vide est descendu à 2 mbar.
Le distillat représenté par les composés sapides et/ou odorants et/ou les composés chimiques résultant de l'altération et de la dégradation des huiles végétales est condensé et refroidi par le condenseur, recueilli dans le pot de recette, puis évacué vers l'extérieur par une pompe.
L'addition de vapeur est alors maintenue pendant 5 h à la température de 210°C.
Le produit est alors refroidi sous courant d'azote, puis soutiré après cassage du vide.
Le bilan massique de l'opération est le suivant :

Distillat de désodorisation mis en oeuvre : 18 kg

Composés sapides et odorants récupérés : 0,23 Kg

Insaponifiable total de tournesol préparé : 3,08 kg

|  |  | DD | Insapo Tot | % récupérés |
|---|---|---|---|---|
| Teneur en Stérols (CPG) | | 7,9% | 40,1% | 90,4 |
| Relatif | Brassicastérol | 0,0% | 0,0% | |
| | Campestérol | 10,6% | 11,7% | |
| | Stigmastérol | 12,0% | 12,9% | |
| | Beta-sitostérol | 55,8% | 57,7% | |
| | Δ7 stigmastérol | 4,8% | 5,1% | |
| Teneur en Tocophérols totaux (HPLC) | | 0,6% | 3,0% | 97,1 |
| Relatif | Alpha-tocophérol | 87,5% | 87,9% | |
| | Gamma-tocophérol | 3,6% | 5,1% | |
| | Beta-tocophérol | 8,8% | 6,9% | |
| Teneur en squalène | | 1,7% | 9,1% | 95,3 |
| Teneur en composés sapides et odorants | | 53,6% | 0,01% | |
| Résidus de pesticides mg/kg | | 12[1] | < LOQ[2] | < 98,8[3] |

(suite)

|  | DD | Insapo Tot | % récupérés |
|---|---|---|---|
| Teneur en insaponifiable | 18,5%[4] | 17,8%[5] | 96,2[6] |

1- Somme des teneurs en pyrimiphos-méthyl, dichlorvos, malathion.
2- Limite quantifiable : 0,05 mg/kg pour chacun des pesticides, pyrimiphos-méthyl, dichlorvos, malathion
3- % de résidus de pesticides éliminés par rapport à la teneur dans le produit de départ : (12 - 0,15) x100 / 12 = 98,8 %.
4- Teneur en insaponifiable dosée dans le distillat de désodorisation.
5- % Rendement en insaponifiable : masse d'insaponifiable total purifié obtenu par rapport à la masse de distillat de désodorisation mise en oeuvre.
6- % Rendement d'extraction : masse d'insaponifiable total purifié obtenu par rapport à la masse théorique dosée dans le distillat de désodorisation mis en oeuvre.

Résultats :

La composition de l'insaponifiable total purifié de tournesol préparé met en évidence les performances du procédé revendiqué, à savoir :

- préparer un insaponifiable total d'huile de tournesol purifié à partir d'un distillat de désodorisation de tournesol préalablement concentré par distillation moléculaire,
- garantir la présence des différentes familles de composants constitutives d'un insaponifiable de tournesol,
- garantir la conservation de la composition relative des différentes familles de composants,
- éliminer les composés sapides et/ou odorants et/ou des composés chimiques résultant de l'altération et de la dégradation des huiles végétales.
- éliminer les traces de résidus de pesticides,
- garantir un taux de récupération important des différents composants, et notamment des stérols et des tocophérols.

Exemple 6

Contre-exemple : extraction de l'insaponifiable total à partir d'huile avec distillation moléculaire (DM).
La préparation de l'insaponifiable total de soja à été réalisée à partir d'une huile de soja raffinée en deux étapes successives :

1ère étape: Préparation d'une fraction concentrée en insaponifiable par distillation moléculaire en utilisant la technologie décrite dans l'exemple 2 et avec les paramètres opératoires suivants :

Débit alimentation : 15,5 kg/h
Vide : $10^{-2}$ mm de Hg
Température de distillation : 230°C
Taux de distillation : 3,9 %

2ème étape : Extraction de l'insaponifiable total par saponification, extraction par solvant, et désodorisation, en utilisant les technologies de saponification et d'extraction décrites dans l'exemple 4.

Bilan matière

Masse d'huile de soja raffinée mise en oeuvre : 198 Kg
Taux de distillation de l'étape 1 : 3,9%
Insaponifiable total de soja extrait à partir du distillat de distillation moléculaire : 7,95%
Insaponifiable total de soja extrait à partir de l'huile de soja : 0,31%
Taux de récupération : 62%
Composition de l'insaponifiable obtenu par DM de l'huile de soja et extraction :

|  | Huile | Insapo Tot | % récupérés |
|---|---|---|---|
| Teneur en Stérols (CPG) | 0,4% | 61,0% | 50,1 |

(suite)

|  |  | Huile | Insapo Tot | % récupérés |
|---|---|---|---|---|
| Relatif | Brassicastérol | 0,3% | 0,3% | |
| | Campestérol | 18,4% | 23,3% | |
| | Stigmastérol | 15,9% | 20,5% | |
| | Beta-sitostérol | 45,4% | 45,9% | |
| Teneur en Tocophérols totaux (HPLC) | | 0,1% | 27,0% | 70,0 |
| Relatif | Alpha-tocophérol | 9,8% | 7,3% | |
| | Gamma-tocophérol | 67,7% | 68,8% | |
| | Delta-tocophérol | 22,6% | 23,7% | |
| Teneur en squalène | | 0,01% | 0,6% | 18,5 |
| Teneur en insaponifiable | | 0,50% | 0,3% | 62,0 |

Conclusion :

La composition de l'insaponifiable total de soja préparé met en évidence les propriétés suivantes du procédé d'extraction à partir de l'huile qui utilise une étape de DM et une étape d'extraction :

-    permet de préparer un insaponifiable total d'huile de soja à partir de l'huile,

-    garantit la présence des différentes familles de composants constitutives d'un insaponifiable de soja,

-    garantit la conservation de la composition relative des différentes familles de composants,

-    ne garantit pas un taux de récupération important des différents composants, et notamment des stérols et du squalène.

Exemple 7 :

Exemple démontrant l'efficacité du procédé selon l'invention pour l'élimination des pesticides.

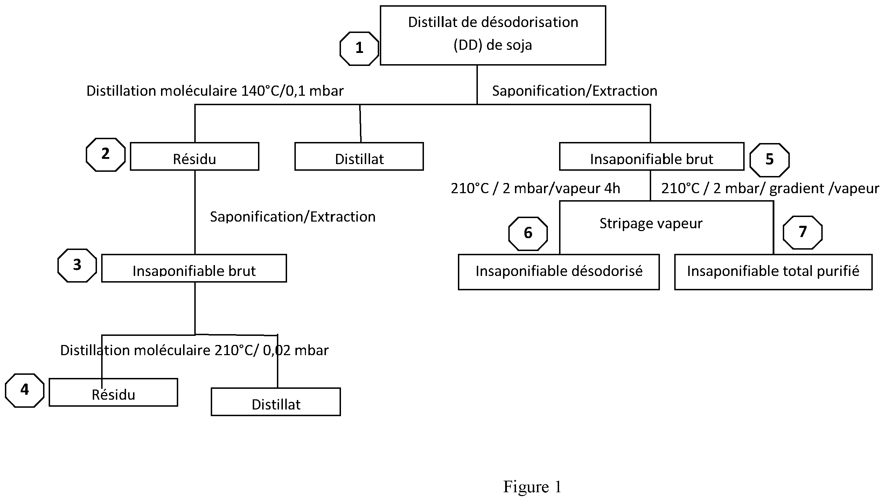

Les techniques de désodorisation et de distillation moléculaire ont été comparées à différentes étapes de la préparation de l'insaponifiable de soja à partir d'un distillat de désodorisation d'huile de soja selon l'invention. La Figure 1 présente ainsi différentes voies d'extraction et de purification de l'insaponifiable de soja.

La voie de gauche de la Figure 1 présente ainsi une étape de distillation moléculaire à partir d'un distillat de désodorisation (DD) d'huile de soja 1, conduisant à l'obtention d'un résidu 2. L'insaponifiable total brut 3 est ensuite extrait à partir du résidu 2 par saponification/extraction. Une distillation moléculaire à température stabilisée à 210°C et 2 mbar a été réalisée sur l'insaponifiable total brut 3 pour conduire au résidu 4.

La voie de droite de la Figure 1 présente ainsi l'obtention de l'insaponifiable total brut 5 de soja à partir d'un distillat de désodorisation (DD) d'huile de soja 1 par saponification/extraction telle que décrite dans l'exemple 4 ci-dessus, conformément à la présente invention. L'insaponifiable total brut 5 est ensuite désodorisé d'une part pour conduire à l'insaponifiable désodorisé 6 par stripage vapeur à température stabilisée à 210°C et 2 mbar. D'autre part, l'insaponifiable total purifié d'huile de soja 7 selon l'invention est obtenu par stripage vapeur sous vide, à partir de l'insaponifiable total brut 5, en utilisant un gradient de température et de vide spécifique conformément à la présente invention, en appliquant ici les conditions décrites dans l'exemple 4 ci-dessus à l'étape (5).

L'évaluation de l'élimination des pesticides a été faite sur l'élimination de l'aldrine et de la dieldrine et les résultats sont présentés dans le tableau 1 ci-dessous.

Tableau 1 :

| échantillon | Code Echantillon (Cf. Fig. 1) | Aldrine mg/kg | Dieldrine mg/kg | Facteur réduction | |
|---|---|---|---|---|---|
| | | | | Aldrine | Dieldrine |
| DD | 1 | 0,41 | 3,40 | | |
| Résidu DD | 2 | 0,20 | 0,72 | 2,05 | 4,7 |
| Insaponifiable brut | 3 | 0,49 | 2,20 | | |
| Résidu insaponifiable après distillation moléculaire | 4 | 0,1 | 1,00 | 4,9 | 2,2 |
| Insaponifiable brut | 5 | 0,44 | 6,86 | | |
| Insaponifiable désodorisé | 6 | Non détecté | 0,30 | >4,4 | 6,7 |
| Insaponifiable total purifié | 7 | Non détecté | Non détecté | >4,4 | >69 |
| Non détecté : < LOQ (0,1 mg/kg) | | | | | |

Conclusion

La technique de distillation moléculaire appliquée avant ou après l'étape d'extraction de l'insaponifiable, présentée sur la voie de gauche de la Figure 1, permet de réduire la teneur en pesticides résiduels, mais n'aboutit pas à une élimination complète.

La désodorisation de l'insaponifiable à une température stabilisée de 210°C conduit à une élimination de la dieldrine qui n'est pas complète.

Par contre, le stripage vapeur appliqué dans les conditions décrites dans l'exemple 4 selon l'invention conduit à une élimination totale de la dieldrine.

Exemple 8 :

Activité biologique

L'articulation est composée de différents tissus, dont les plus importants impliqués dans les pathologies articulaires telles que l'arthrose, sont le cartilage, la membrane synoviale et l'os sous-chondral.

Le cartilage articulaire est un tissu conjonctif qui, au sein de l'articulation, est en contact par sa couche la plus profonde avec l'os sous-chondral et par sa couche la plus superficielle avec le fluide synovial. Les propriétés uniques du cartilage sont corrélées à la composition et la structure de sa matrice extracellulaire, qui est composée majoritairement d'une forte proportion de protéoglycanes tels que l'agrécane, piégés dans un réseau dense de fibres de collagène et d'une grande quantité d'eau. Le cartilage articulaire comprend des cellules, chondrocytes, responsables de la synthèse et du maintien de la matrice extracellulaire du cartilage. Dans le cartilage normal, il y a un état d'équilibre établi entre la synthèse et la dégradation de cette matrice extracellulaire.

Durant l'arthrose, l'homéostasie du cartilage est altérée via une perturbation du métabolisme du chondrocyte dans le sens d'une augmentation du catabolisme et une réduction de l'anabolisme.

Une des principales stratégies pour les traitements anti-arthrosiques est d'inhiber ou d'arrêter la destruction du cartilage en :

- inhibant les facteurs cataboliques comme les médiateurs inflammatoires,
- stimulant les mécanismes de réparation du cartilage, en augmentant la synthèse des composants majeurs de la matrice.

Les effets de différentes fractions d'insaponifiables à potentialité anti-arthrosique ont été evalués sur le métabolisme de chondrocytes de cartilage humains arthrosiques cultivés en billes d'alginate durant 12 jours. Différents aspects du métabolisme des chondrocytes ont été étudiés afin de dresser le profil pharmacologique de ces composés. Leurs effets ont été étudiés sur la production de cibles anaboliques indispensables à la reconstruction de la matrice cartilagineuse comme l'agrécane (AGG) et le TGFbetal (TGFb1) et de cibles cataboliques impliquées dans la destruction du cartilage comme l'interleukine 6 (IL-6) et le monoxyde d'azote (NO).

Le modèle de culture de chondrocytes humains en bille d'alginate utilisé dans cette étude permet de mimer l'environnement du chondrocyte dans le cartilage.

L'agrécane est le protéoglycane majoritaire et spécifique du cartilage hyalin auquel il confère une grande résistance aux compressions. De plus, dans l'arthrose, l'agrécane est déficitaire et représente une cible thérapeutique potentielle.

Le facteur de croissance TGFbeta 1 « transforming growth factor » beta joue un rôle clé dans la stimulation et la réparation des composants de la matrice extracellulaire. Le TGFbeta est exprimé par les chondrocytes et est capable de stimuler l'expression de molécules matricielles. De plus, le TGFbeta est connu aussi pour exercer des effets antagonistes à l'IL-lbeta. Ainsi, stimuler le TGFbeta peut avoir des effets bénéfiques dans la prévention de l'érosion progressive du cartilage survenant dans l'arthrose.

L'IL-6 a été mesurée car il s'agit d'une cytokine de référence pour évaluer la réponse inflammatoire des cellules. Par ailleurs, l'IL-6 diminue l'expression du collagène de type II et de l'agrécane dans des chondrocytes.

Le monoxyde d'azote est une forme activée de l'oxygène ubiquitaire, impliqué dans la réponse inflammatoire et le stress oxydant. Les médiateurs du stress oxydant jouent un rôle non négligeable dans la physiopathologie de l'arthrose. En particulier, le monoxyde d'azote est décrit comme contribuant au développement des lésions arthrosiques en limitant les macromolécules matricielles du cartilage d'une part et en induisant la mort cellulaire des chondrocytes d'autre part.

Les différentes fractions testées sont les suivantes :

- Fraction stérolique purifiée d'insaponifiable de soja : teneur en stérols > 95%
- Fraction totale d'insaponifiable d'avocat furanique = Fraction furanique d'insaponifiable d'avocat + Fraction alcools trihydroxylés d'insaponifiable d'avocat + Fraction stérolique d'insaponifiable d'avocat + Fraction squalène
- Insaponifiable total de soja purifié selon la présente invention + Fraction totale d'insaponifiable d'avocat furanique

Les résultats ont été exprimés en quantité d'agrécane (en ng), de TGFbetal (en pg), d'IL-6 (en pg) et de NO2+NO3 (en nmoles) par $\mu$g d'ADN. La moyenne et l'écart-type réduit (SEM) ont été calculés pour chaque condition de culture. Les moyennes obtenues ont été comparées par le test non paramétrique U de Mann Whitney. Le pourcentage d'activité des composés a été calculé par rapport au témoin (cellules non traitées par les composés). Les résultats ont été pondérés en fonction de la significativité statistique. Ainsi : NS = pas d'activité ; $P < 0.05$ = + ; $P < 0.01$ = ++ ; $P < 0.001$ = +++.

Effet des fractions sur la production d'agrécane

| Conditions | AGG ng/ $\mu$g ADN | écart-type | % par rapport au témoin | Significa tivité |
|---|---|---|---|---|
| Témoin | 9804,59 | 601,07 | | |
| Insaponifiable total de soja purifié + Fraction totale d'insaponifiable d'avocat furanique | 11568,00 | 522,58 | 118% | P<0.01 = ++ |
| Fraction totale d'insaponifiable d'avocat furanique + Fraction stérolique purifiée d'insaponifiable de soja | 9517,00 | 1215,57 | 97% | NS = 0 |

Effet des fractions sur la production de TGFbetal

| Conditions | TGFb1 pg/ $\mu$g ADN | écart-type | % par rapport au témoin | Significa tivité |
|---|---|---|---|---|
| Témoin | 81,47 | 7,35 | | |
| Insaponifiable total de soja purifié + Fraction totale d'insaponifiable d'avocat furanique | 106,77 ' | 9,29 | 131% | P<0.01 = ++ |
| Fraction totale d'insaponifiable d'avocat furanique + Fraction stérolique purifiée d'insaponifiable de soja | 96,59 | 7,07 | 118% | NS = 0 |

Effet des fractions sur la production d'IL-6

| Conditions | IL-6 pg/$\mu$g ADN | écart-type | % par rapport au témoin | Significa tivité |
|---|---|---|---|---|
| Témoin | 11104,73 | 905,06 | | |
| Insaponifiable total de soja purifié + Fraction totale d'insaponifiable d'avocat furanique | 7511,13 | 705,82 | 68% | P<0.001 = +++ |
| Fraction totale d'insaponifiable d'avocat furanique + Fraction stérolique purifiée d'insaponifiable de soja | 9968,84 | 1365,94 | 90% | NS = 0 |

Effet des fractions sur la production de NO

| Conditions | NO2+N03 nmole/ $\mu$g ADN | écart-type | % par rapport au témoin | Significa tivité |
|---|---|---|---|---|
| Témoin | 64,82 | 0,89 | | |
| Insaponifiable total de soja purifié + Fraction totale d'insaponifiable d'avocat furanique | 54,74 ' | 0,91 | 84% | P<0.01 = ++ |
| Fraction totale d'insaponifiable d'avocat furanique + Fraction stérolique purifiée d'insaponifiable de soja | 58,21 | 4,43 | 90% | NS = 0 |

En conclusion :

Le mélange insaponifiable total de soja purifié + la fraction totale d'insaponifiable d'avocat furanique stimule l'agrécane et le TGFbetal (++) et inhibe l'IL-6 et le NO (+++) et ledit mélange a une activité supérieure au mélange de la fraction totale d'insaponifiable d'avocat furanique + la fraction stérolique purifiée d'insaponifiable de soja, qui ne stimule ni l'agrécane, ni le TGFbetal, et n'inhibe ni l'IL-6, ni le NO.

[0216] Par conséquent, seule l'association de l'insaponifiable total de soja purifié selon le procédé de la présente invention en mélange avec la fraction totale d'insaponifiable d'avocat, avantageusement d'insaponifiable furanique, a un spectre d'activité important sur le métabolisme du chondrocyte et présente donc un intérêt dans la prise en charge de l'arthrose.

**Revendications**

**1.** Utilisation d'au moins un co-produit de l'industrie du raffinage des huiles végétales, tel qu'un distillat de désodorisation d'huiles végétales et/ou un condensat de raffinage physique pour obtenir un insaponifiable total purifié d'huile végétale comprenant tous les constituants et familles de constituants naturellement présents dans l'insaponifiable de ladite huile végétale et débarrassé des impuretés initialement présentes dans ledit co-produit, telles que des composés sapides et/ou odorants et/ou des composés chimiques résultant de l'altération et de la dégradation des huiles végétales, **caractérisée en ce que** l'insaponifiable total purifié d'huile végétale est obtenu par au moins une étape de saponification et par élimination des composés sapides et/ou odorants et/ou les composés chimiques résultant de l'altération et de la dégradation des huiles végétales par au moins une étape d'entraînement par stripage par un gaz vecteur avec un gradient de température et de vide, avantageusement à une température croissante de 80°C à 250°C, typiquement de 145°C à 210°C, et une progression de 0,5 à 2°C/min, typiquement de 1°C/min, et sous un vide décroissant de 50 à 1 mbar, typiquement de 30 à 2 mbar, et une progression de 0,1 à 10 mbar/min, typiquement de 0,5 à 5 mbar/min.

**2.** Utilisation selon la revendication 1, **caractérisée en ce que** l'insaponifiable total purifié est débarrassé des résidus de produits phytosanitaires du type pesticides, avantageusement par au moins une étape de stripage par un gaz vecteur sous vide, typiquement à une température comprise entre 180°C et 250 °C et sous un vide de 1 à 5 mbar.

**3.** Utilisation selon la revendication 1 ou 2, **caractérisée en ce que** l'huile végétale est l'huile de soja.

**4.** Procédé d'obtention d'un insaponifiable total purifié d'huile végétale à partir d'au moins un co-produit de l'industrie du raffinage des huiles végétales, ledit insaponifiable total purifié comprenant tous les constituants et familles de constituants naturellement présents dans l'insaponifiable de ladite huile végétale et étant débarrassé des impuretés initialement présentes dans ledit co-produit, comprenant les étapes successives suivantes :

Optionnellement, étape préalable de concentration en insaponifiable du co-produit de l'industrie du raffinage des huiles végétales, avantageusement par distillation moléculaire,

(1) Saponification en milieu hydro-alcoolique par une base alcaline du type potasse,
(2) Extraction liquide/liquide par un solvant organique, avantageusement choisi dans le groupe constitué par les alcanes, les solvants chlorés tels que le 1,2-dichloroéthane (DCE), les solvants aromatiques fluorés, les tert-butyl éthers, les solvants comprenant un atome de silicium, le MeTHF, et leurs mélanges,
(3) Lavage à l'eau de la solution organique extraite, avantageusement par extraction liquide/liquide,
(4) Evaporation du solvant organique, puis
(5) Stripage par un gaz vecteur sous vide, tel que la vapeur sèche ou l'azote, afin d'obtenir un insaponifiable total purifié d'huile végétale,

**caractérisé en ce que** le stripage (5) par un gaz vecteur sous vide comprend au moins une étape (b) d'entraînement avec un gradient de température et de vide, avantageusement à une température croissante de 80°C à 250°C, typiquement de 145°C à 210°C, et une progression de 0,5 à 2°C/min, typiquement de 1°C/min, et sous un vide décroissant de 50 à 1 mbar, typiquement de 30 à 2 mbar, et une progression de 0,1 à 10 mbar/min, typiquement de 0,5 à 5 mbar/min, afin d'éliminer les composés sapides et/ou odorants et/ou les composés chimiques résultant de l'altération et de la dégradation des huiles végétales.

**5.** Procédé d'obtention d'un insaponifiable total purifié d'huile végétale selon la revendication 4, **caractérisé en ce que** le stripage par un gaz vecteur sous vide (5) comprend une étape préliminaire (a) d'élimination du solvant organique résiduel, avantageusement à une température comprise entre 80°C et 145 °C et sous un vide de 200 à 30 mbar.

**6.** Procédé d'obtention d'un insaponifiable total purifié d'huile végétale selon la revendication 4 ou 5, **caractérisé en ce que** le stripage (5) par un gaz vecteur sous vide comprend en outre une étape (c) d'élimination des résidus de produits phytosanitaires du type pesticides, avantageusement à une température comprise entre 180°C et 250°C et sous un vide de 1 à 5 mbar, et encore plus avantageusement à une température de 210°C sous un vide de 2 mbar.

**Patentansprüche**

**1.** Verwendung wenigstens eines Nebenerzeugnisses der Industrie der Raffinerie von Pflanzenölen, wie z. B. ein Desodorisierungsdestillat aus Pflanzenölen und / oder ein Kondensat aus physikalischem Raffinieren, um ein unverseifbares, vollständige gereinigtes Pflanzenöl zu erhalten, das alle Bestandteile und Familien von Bestandteilen umfasst, die im Unverseifbaren des genannten Pflanzenöls natürlicherweise enthalten, und von den ursprünglich in dem genannten Nebenerzeugnis enthaltenen Unreinheiten befreit ist, wie z. B. Geschmacks- und / oder Geruchsbestandteile und / oder chemische Bestandteile, die aus dem chemischen Angriff oder dem Abbau von Pflanzenölen resultieren, **dadurch gekennzeichnet, dass** das unverseifbare, vollständige gereinigte Pflanzenöl durch wenigstens eine Verseifungsschritt und durch den Austrag der Geschmacks- und / oder Geruchsbestandteile und / oder die chemischen Bestandteile erhalten ist, die aus dem chemischen Angriff und dem Abbau der Pflanzenöle durch wenigstens einen Antriebsschritt per Austreiben durch ein Trägergas mit einem Temperatur- und Vakuumgradienten, vorteilhaft bei einer ansteigenden Temperatur von 80° C auf 250° C, typischerweise von 145° C auf 210° C und einer Progression von 0,5 bis 2° C / Min., typischerweise von 1° C / Min, und unter einem absteigenden Vakuum von 50 bis 1 mbar, typischerweise von 30 bis 2 mbar und einer Progression von 0,1 bis 10 mbar / Min., typischerweise von 0,5 bis 5 mbar / Min. resultieren.

**2.** Verwendung gemäß Anspruch 1, **dadurch gekennzeichnet, dass** das vollständige, gereinigte Unverseifbare von Rückständen von Pflanzenschutzprodukten vom Typ Schädlingsbekämpfungsmittel, vorteilhaft durch wenigstens einen Austreibschritt durch ein Trägergas unter Vakuum, typischerweise bei einer zwischen 180° C und 250° C inbegriffenen Temperatur und unter einem Vakuum von 1 bis 5 mbar befreit ist.

3. Verwendung gemäß Anspruch 1 oder 2, **dadurch gekennzeichnet, dass** das Pflanzenöl Sojaöl ist.

4. Verfahren zum Erhalt eines unverseifbaren, vollständigen, gereinigten Pflanzenöls ausgehend von wenigstens einem Nebenerzeugnis der Industrie der Raffinerie von Pflanzenölen, wobei das genannte vollständige, gereinigte Unverseifbare alle Bestandteile und Familien von Bestandteilen umfasst, die natürlicherweise in dem Unverseifbaren des genannten Pflanzenöls vorhanden sind, und von ursprünglich in dem genannten Nebenerzeugnis vorhandenen Unreinheiten befreit sind, umfassend die folgenden Schritte:

Optional vorheriger Konzentrationsschritt in Unverseifbares des Nebenerzeugnisses der Industrie der Raffinerie von Pflanzenölen, vorteilhaft per Molekulardestillation;

(1) Verseifung im hydro-alkoholischen Milieu durch eine alkalische Basis vom Typ Kaliumchlorid,
(2) Extraktion Flüssigkeit / Flüssigkeit durch ein organisches Lösungsmittel, das vorteilhaft aus der Gruppe ausgewählt ist, die aus Alkanen, gechlorten Lösungsmitteln, wie z. B. 1,2-Dichlorethan (DEC), fluorierten aromatischen Lösungsmitteln, tert-Butylethern, ein Siliziumatom umfassende Lösungsmittel, MeTHF und deren Mischungen gebildet ist,
(3) Waschen der vorteilhaft per Extraktion Flüssigkeit / Flüssigkeit extrahierten, organischen Lösung in Wasser,
(4) Verdampfen der organischen Lösung, dann
(5) Austreiben durch ein Trägergas unter Vakuum, wie z. B. trockenem Dampf oder Stickstoff, um ein unverseifbares, vollständiges, gereinigtes Pflanzenöl zu erhalten,

**dadurch gekennzeichnet, dass** das Austreiben (5) durch ein Trägergas unter Vakuum wenigstens einen Antriebsschritt (b) mit einem Temperatur- und Vakuumgradienten, vorteilhaft bei einer ansteigenden Temperatur von 80° C bis 250° C, typischerweise von 145° C bis 210° C und einer Progression von 0,5 bis 2° C / Min., typischerweise von 1° C / Min. und unter einer absteigenden Vakuum von 50 bis 1 mbar, typischerweise von 30 bis 2 mbar und einer Progression von 0,1 bis 10 mbar / Min, typischerweise von 0,5 bis 5 mbar / Min. umfasst, um die Geschmacks- und / oder Geruchsbestandteile und / oder die chemischen Bestandteile auszutragen, die aus dem chemischen Angriff und dem Abbau der Pflanzenöle resultieren.

5. Verfahren zum Erhalt eines unverseifbaren, vollständigen, gereinigten Pflanzenöls gemäß Anspruch 4, **dadurch gekennzeichnet, dass** das Austreiben durch ein Trägergas unter Vakuum (5) einen einleitenden Austragsschritt (a) des restlichen organischen Lösungsmittels, vorteilhaft bei einer zwischen 80° C und 145° C inbegriffenen Temperatur und unter einem Vakuum von 200 bis 30 mbar, umfasst.

6. Verfahren zum Erhalt eines unverseifbaren, vollständigen, gereinigten Pflanzenöls gemäß Anspruch 4 oder 5, **dadurch gekennzeichnet, dass** das Austreiben durch ein Trägergas unter Vakuum darüber hinaus einen Austragsschritt (c) der Rückstände von Pflanzenschutzprodukten vom Typ Schädlingsbekämpfungsmittel, vorteilhaft bei einer zwischen 180° C und 250° C inbegriffenen Temperatur und unter Vakuum von 1 bis 5 mbar und weiter vorteilhaft bei einer Temperatur von 210° C unter einem Vakuum von 2 mbar umfasst.

**Claims**

1. Use of at least one vegetable oil refining industry by-product, such as a deodoriser distillate of vegetable oils and/or a physical refining condensate, to obtain a purified total unsaponifiable of vegetable oil comprising all the constituents and families of constituents initially present in the unsaponifiable of said vegetable oil and cleared of the impurities initially present in said by-product, such as sapid and/or odorous compounds and/or chemical compounds resulting from the deterioration and the degradation of vegetable oils, **characterised in that** the purified total unsaponifiable of vegetable oil is obtained by at least one step of saponification and by eliminating sapid and/or odorous compounds and/or chemical compounds resulting from the deterioration and the degradation of vegetable oils by at least one step of entrainment by carrier gas stripping with a temperature and vacuum gradient, advantageously at a temperature increasing from 80°C to 250°C, typically from 145°C to 210°C, and a progression of 0.5°C/min to 2°C/min, typically of 1°C/min, and under a vacuum decreasing from 50 mbar to 1 mbar, typically from 30 mbar to 2 mbar, and a progression of 0.1 mbar/min to 10 mbar/min, typically from 0.5 mbar/min to 5 mbar/min.

2. The use according to claim 1, **characterised in that** the purified total unsaponifiable is cleared of phytosanitary product residues such as pesticides, advantageously by at least one step of carrier gas stripping under vacuum, typically at a temperature between 180°C and 250°C and under a vacuum of 1 mbar to 5 mbar.

3. The use according to claim 1 or 2, **characterised in that** the vegetable oil is soya oil.

4. A method for obtaining a purified total unsaponifiable of vegetable oil from at least one vegetable oil refining industry by-product, said purified total unsaponifiable comprising all the constituents and families of constituents initially present in the unsaponifiable of said vegetable oil and being cleared of the impurities initially present in said by-product, comprising the following successive steps:

> optionally, a preliminary step of concentration in unsaponifiable of the vegetable oil refining industry by-product, advantageously by molecular distillation,
>
>> (1) saponification in hydroalcoholic medium by a potash-type alkaline base,
>> (2) liquid-liquid extraction by an organic solvent, advantageously selected from the group consisting of alkanes, chlorinated solvents such as 1,2-dichloroethane (DCE), fluorinated aromatic solvents, tert-butyl ethers, solvents comprising a silicon atom, MeTHF, and mixtures thereof,
>> (3) washing with water of the organic solution extracted, advantageously by liquid/liquid extraction,
>> (4) evaporation of the organic solvent, then
>> (5) stripping by a carrier gas under vacuum, such as dry steam or nitrogen, in order to obtain a purified total unsaponifiable of vegetable oil,
>
> **characterised in that** the carrier gas stripping under vacuum (5) comprises at least one step (b) of entrainment with a temperature and vacuum gradient, advantageously at a temperature increasing from 80°C to 250°C, typically from 145°C to 210°C, and a progression of 0.5°C/min to 2°C/min, typically of 1°C/min, and under a vacuum decreasing from 50 mbar to 1 mbar, typically from 30 mbar to 2 mbar, and a progression of 0.1 mbar/min to 10 mbar/min, typically from 0.5 mbar/min to 5 mbar/min, in order to eliminate sapid and/or odorous compounds and/or chemical compounds resulting from the deterioration and the degradation of vegetable oils.

5. The method for obtaining a purified total unsaponifiable of vegetable oil according to claim 4, **characterised in that** the carrier gas stripping under vacuum (5) comprises a preliminary step (a) of elimination of residual organic solvent, advantageously at a temperature between 80°C and 145°C and under a vacuum of 200 mbar to 30 mbar.

6. The method for obtaining a purified total unsaponifiable of vegetable oil according to claim 4 or 5, **characterised in that** the carrier gas stripping under vacuum (5) further comprises a step (c) of elimination of phytosanitary product residues such as pesticides, advantageously at a temperature between 180°C and 250°C and under a vacuum of 1 mbar to 5 mbar, and even more advantageously at a temperature of 210°C under a vacuum of 2 mbar.

Figure 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2803598
- WO 2010004193 A
- US 20040030166 A

**Littérature non-brevet citée dans la description**

- **SARKADI.** *J.A.O.C.S.,* 1958, vol. 35, 472-475